# EUROPEAN PATENT SPECIFICATION

(11) **EP 2 844 731 B1**
(45) Date of publication and mention of the grant of the patent: **31.01.2018**
(21) Application number: 13747610.7
(22) Date of filing: 01.08.2013
(51) Int. Cl.: C12N 15/80, C12N 1/14, C12N 9/00, C12N 9/88, C12P 21/00

(54) **FUNGAL CELLS THAT PRODUCE DECREASED AMOUNTS OF PEPTAIBOLS**
VERRINGERTE PEPTAIBOLMENGEN PRODUZIERENDE PILZE
CELLULES FONGIQUES PRODUISANT DES QUANTITÉS RÉDUITES DE PEPTAIBOLS

(30) Priority: 06.08.2012 US 201261680057 P; 08.08.2012 US 201261681071 P
(43) Date of publication of application: 11.03.2015
(60) Divisional application: 17206165.7
(73) Proprietor: Danisco US Inc., Palo Alto, California 94304 (US)
(72) Inventor: WARD, Michael, Palo Alto, California 94304 (US)
(74) Representative: Kiddle, Simon John
(86) International application number: PCT/US2013/053177
(87) International publication number: WO 2014/025605

(56) References cited:
- WEI XIANYING ET AL: "Multiple non-ribosomal peptide synthetase genes determine peptaibol synthesis in Trichoderma virens", CANADIAN JOURNAL OF MICROBIOLOGY, NRC RESEARCH PRESS, CA, vol. 51, no. 5, 1 May 2005 (2005-05-01), pages 423-429, XP009173578, ISSN: 0008-4166
- ANDRÃ CR SCHUSTER ET AL: "A versatile toolkit for high throughput functional genomics with Trichoderma reesei", BIOTECHNOLOGY FOR BIOFUELS, BIOMED CENTRAL LTD, GB, vol. 5, no. 1, 2 January 2012 (2012-01-02) , page 1, XP021094241, ISSN: 1754-6834, DOI: 10.1186/1754-6834-5-1
- LUMSDEN R D ET AL: "BIOLOGICAL CONTROL OF DAMPING-OFF CAUSED BY PYTHIUM-ULTIMUM AND RHIZOCTONIA-SOLANI WITH GLIOCLADIUM-VIRENS IN SOILLESS MIX", PHYTOPATHOLOGY, vol. 79, no. 3, 1989, pages 361-366, XP002715303, ISSN: 0031-949X
- P. K. MUKHERJEE ET AL: "Secondary metabolism in Trichoderma - a genomic perspective", MICROBIOLOGY, vol. 158, no. 1, 13 October 2011 (2011-10-13), pages 35-45, XP055085056, ISSN: 1350-0872, DOI: 10.1099/mic.0.053629-0
- R D Lumsden ET AL: "Disease Control and Pest Management Biological Control of Damping-Off Caused by Pythium ultimum and Rhizoctonia solani with Gliocladium virens in Soilless Mix", , 1 January 1989 (1989-01-01), pages 361-366, XP055085109, Retrieved from the Internet: URL:http://www.apsnet.org/publications/phy topathology/backissues/Documents/1989Artic les/Phyto79n03_361.PDF [retrieved on 2013-10-23]

## Description

### BACKGROUND

Peptaibols are linear peptide antibiotics produced in a variety of Ascomycetes fungal cells by non-ribosomal peptide synthetases (NRPS). Peptaibols are typically less than 20 residues in length, with C-terminal alcohol residues, non-standard amino acid residues, including α-aminoisobutyric acid (Aib) and isovaline (Iva), and the imino acid, hydroxyproline (Hyp). *Trichoderma* (teleomorph *Hypocrea*) and related genera) are known to produce significant amounts of peptaibols, which have been shown to exhibit antibacterial, antifungal, and even anti-cancer properties. In particular, *T. reesei, T. atroviride,* and *T. virens* produce 11-and 14-residue peptaibols, which can used as biological control agents for the management of plant pathogens. Peptaibols are produced by Ascomycetes spp. fungal cells grown on solid medium, as opposed to liquid medium, and have heretofore not been considered important to the use of Ascomycetes fungal cells as hosts for production of industrially important polypeptides in submerged culture.

NRPSs are proteins that are capable of enzymatically assembling small peptides. NRPSs have modular organization, wherein each module consists of about 1100 amino-acid residues, which form, at minimum, an adenylation (A) domain, a thiolation (T) domain, and a condensation (C) domain, which together catalyze the incorporation of a single amino acid residue into an emerging peptaibol.

The number of modules in an NRPS determines the maximum number of residues in the resulting peptaibols. For example, an 18-module NRPS generate a maximum 18-residue peptaibol. However, NRPS having a given number of modules can also generate shorter peptaibols through module skipping. For example, certain 14-moldule NRPS have been shown do produce at least 11 and 14-residue peptaibols. As a result of module skipping, a single NRPS protein can be responsible for generating an entire class of peptaibols in a cell. Particular, previously-described NRPS in *Trichoderma* spp. include Tex1 and Tex2. Disruption of the *tex1* gene prevents the synthesis of all 18-residue peptaibols. Disruption of the *tex2* gene prevents the synthesis of 14 and 11-residue peptaibols. *Trichoderma* spp. peptaibol and NRPS are described in detail in, for example: Degenkolb, T. et al. (2012) Chemistry & Biodiversity 9:499-535; Wiest, A. et al. (2002) J. Biol. Chem. 277:20862-68; Mukherjee, P. et al. (2011) J. Biol. Chem. 286:4544-54; Viterbo, A. et al. (2007) Molecular Plant Pathology 8:737-46; and Wei, X. et al. (2005) Can. J. Microbiol. 51:423-9.

### SUMMARY

Described are compositions and methods relating to biomass conversion using fungal cells that produce decreased amounts of peptaibols. The present compositions and methods relate to variant Ascomycetes fungal cells that are genetically-modified to reduce the production of peptaibols, particularly when grown on substrates containing insoluble materials.

The present invention provides methods as defined in the claims.

Aspects and embodiments of the methods are set forth in the following separately numbered paragraphs.
1. In one aspect, a method for producing a protein of interest in Ascomycete fungal cells grown in a medium that includes insoluble material is provided, comprising: expressing the protein of interest in variant Ascomycete fungal cells comprising a genetic modification that disrupts at least one non-ribosomal peptide synthetase (NRPS), wherein the variant cells produce a reduced amount of peptaibols relative to the amount of peptaibols produced by equivalent, non-modified Ascomycete fungal cells when grown in a medium comprising insoluble material.
2. In some embodiments of the method of paragraph 1, the medium including insoluble material comprises a carbon source provided in the form of a semi-solid suspension.
3. In some embodiments of the method of any of the preceding numbered paragraphs, the medium is a biomass substrate.
4. In some embodiments of the method of any of the preceding numbered paragraphs, the genetic modification to reduce the production of peptaibols is the disruption of at least one non-ribosomal peptide synthetase (NRPS) gene without the introduction of foreign DNA.
5. In some embodiments of the method of any of the preceding numbered paragraphs, the genetic modification to reduce the production of peptaibols is the complete or partial deletion of at least one non-ribosomal peptide synthetase (NRPS) gene.
6. In some embodiments of the method of any of the preceding numbered paragraphs, the non-ribosomal peptide synthetase (NRPS) gene is *tex1* or *tex2.*
7. In some embodiments of the method of any of the preceding numbered paragraphs, the Ascomycete fungal cells are filamentous fungi cells in the subphylum Pezizomycotina. In some embodiments of the method of any of the preceding numbered paragraphs, the Ascomycete fungal cells are filamentous fungi cells in the order Hypocreales.
8. In some embodiments of the method of any of the preceding numbered paragraphs, the Ascomycete fungal cells are a *Trichoderma* spp. Cells. In some embodiments of the method of any of the preceding numbered paragraphs, the Ascomycete fungal cells are a Hypocrea spp. cells
9. In some embodiments of the method of any of the preceding numbered paragraphs, the Ascomycete fungal cells are *Trichoderma reesei* cells.
10. In some embodiments of the method of any of the preceding numbered paragraphs, the protein of interest is a secreted protein.
11. In another aspect, a method for producing a protein of interest in Ascomycete fungal cells is provided, comprising: expressing the protein of interest in variant Ascomycete fungal cells comprising a genetic modification that reduces the production of peptaibols relative to the amount of peptaibols produced by equivalent, non-modified Ascomycete fungal cells; wherein the genetic modification disrupts at least one non-ribosomal peptide synthase (NRPS); wherein the reduced production of peptaibols results in improved growth of the variant Ascomycete fungal cells compared to the non-modified Ascomycete fungal cells; improved growth of a second microbial organism grown in the presence of the variant Ascomycete fungal cells compared to the growth of the second microbial organism grown in the presence of equivalent non-modified Ascomycete fungal cells; or improved growth of the second microbial organism grown in the presence of the protein of interest produced by the variant Ascomycete fungal cells, compared to growth of the second microbial organism grown in the presence of a protein of interest produced by equivalent non-modified Ascomycete fungal cells.
12. In some embodiments of the method of paragraph 11, the variant Ascomycete fungal cells are grown on a carbon source that includes insoluble material.
13. In some embodiments of the method of paragraph 11, the variant Ascomycete fungal cells are grown on a carbon source provided in the form of a semi-solid suspension.
16. In some embodiments of the method of any of preceding numbered paragraphs 11-13, the genetic modification to reduce the production of peptaibols is the disruption of at least one non-ribosomal peptide synthetase (NRPS) gene without the introduction of foreign DNA.
17. In some embodiments of the method of any of preceding numbered paragraphs 11-16, the genetic modification to reduce the production of peptaibols is the complete or partial deletion of at least one non-ribosomal peptide synthetase (NRPS) gene.
18. In some embodiments of the method of any of preceding numbered paragraphs 11-17, the non-ribosomal peptide synthetase (NRPS) gene is *tex1* or *tex2.*
19. In some embodiments of the method of any of preceding numbered paragraphs 11-18, the Ascomycete fungal cells are filamentous fungi cells in the subphylum Pezizomycotina. In some embodiments of the method of any of the preceding numbered paragraphs 11-18, the Ascomycete fungal cells are filamentous fungi cells in the order Hypocreales.
20. In some embodiments of the method of any of preceding numbered paragraphs 11-19, the Ascomycete fungal cells are a *Trichoderma* spp. cells. In some embodiments of the method of any of preceding numbered paragraphs 11-19, the Ascomycete fungal cells are a Hypocrea spp. cells
21. In some embodiments of the method of any of preceding numbered paragraphs 11-20, the Ascomycete fungal cells are *Trichoderma reesei* cells.
22. In some embodiments of the method of any of preceding numbered paragraphs 11-21, the protein of interest is a secreted protein.
23. In another aspect, a method for improving the conversion of a biomass substrate into a fermented end-product, or intermediate, thereof, is provided, comprising contacting the biomass substrate with variant Ascomycete fungal cells producing enzymes for hydrolyzing the biomass substrate into sugars for use by cells of a second, fermenting organism capable of fermenting the sugars into end products, or intermediates, thereof, the variant Ascomycete fungal cells comprising a genetic modification that disrupts at least one non-ribosomal peptide synthase (NRPS) to reduce the production of peptaibols relative to the amount produced by equivalent, non-modified Ascomycete fungal cells, wherein the reduced production of peptaibols by the variant Ascomycete fungal cells improves the yield of end products, or intermediates, thereof, produced by the cells of the second organism compared to the amount of end products, or intermediates, thereof, produced by the cells of the second organism from fermentable sugars produced the equivalent, non-modified Ascomycete cells.
26. The method of numbered paragraph 23, wherein the genetic modification to reduce the production of peptaibols is the disruption of at least one non-ribosomal peptide synthetase (NRPS) gene without the introduction of foreign DNA.
27. The method of any of numbered paragraphs 23-26, wherein the genetic modification to reduce the production of peptaibols is the complete or partial deletion of at least one non-ribosomal peptide synthetase (NRPS) gene.
28. The method of any of numbered paragraphs 23-27, wherein the non-ribosomal peptide synthetase (NRPS) gene is *tex1* or *tex2.*
29. The method of any of numbered paragraphs 23-28, wherein the Ascomycete fungal cells are filamentous fungi cells in the subphylum Pezizomycotina. In some embodiments of the method of any of the preceding numbered paragraphs 23-28, the Ascomycete fungal cells are filamentous fungi cells in the order Hypocreales.
30. The method of any of numbered paragraphs 23-29, wherein the Ascomycete fungal cells are *Trichoderma* spp. cells. The method of any of numbered paragraphs 23-29, wherein the Ascomycete fungal cells are Hypocrea spp. cells.
31. The method of any of numbered paragraphs 23-30, wherein the Ascomycete fungal cells are *Trichoderma reesei* cells.
32. The method of any of numbered paragraphs 23-31, wherein the fermenting organism is a bacteria or yeast.
33. The method of any of numbered paragraphs 23-32, wherein the fermenting organism is a yeast.
34. The method of any of numbered paragraphs 23-32, wherein the fermenting organism is selected from the group consisting of a *Saccharomyces* spp. and a *Zymomonas* spp.
35. The method of any of numbered paragraphs 23-34, wherein the fermenting organism capable of fermenting the sugars is genetically modified to be capable of utilizing both six-carbon sugars and five-carbon sugars.

Also described herein are proteins of interest and variant fungal cells as set out in the following numbered paragraphs:
36. A protein of interest produced by the method of any of numbered paragraphs 1-22.
37. An end product or intermediate produced by the method of any of numbered paragraphs 23-35.
38. Variant, genetically-modified Ascomycete fungal cells for use according to the method of any of the numbered paragraphs.
39. In another aspect, variant Ascomycete fungal cells are provided, comprising a genetic modification that reduces the production of peptaibols relative to the amount produced by an equivalent, non-modified Ascomycete, while not adversely affecting the expression and/or secretion of polypeptides.
40. The variant Ascomycete fungal cells of numbered paragraph 39, wherein the genetic modification that reduces the production of peptaibols is a genetic modification that reduces the production of at least one non-ribosomal peptide synthetase (NRPS).
41. The variant Ascomycetes fungal cells of numbered paragraphs 39 or 40, wherein the genetic modification that reduces the production of peptaibols is the disruption of at least one non-ribosomal peptide synthetase (NRPS) gene.
42. The variant Ascomycete fungal cells of any of any of numbered paragraphs 39-41, wherein the genetic modification that reduces the production of peptaibols is the complete or partial deletion of at least one non-ribosomal peptide synthetase (NRPS) gene.
43. The variant Ascomycete fungal cells of any of numbered paragraphs 40-42, wherein the non-ribosomal peptide synthetase (NRPS) gene is *tex1* or *tex2.*
44. The variant Ascomycete fungal cells of any of numbered paragraphs 39-43, wherein the Ascomycetes fungal cells are filamentous fungi cells in the subphylum Pezizomycotina. The variant Ascomycete fungal cells of any of numbered paragraphs 39-43, wherein the Ascomycetes fungal cells are filamentous fungi cells in the order Hypocreales.
45. The variant Ascomycete fungal cells of any of numbered paragraphs 39-44, wherein the Ascomycete fungal cells are *Trichoderma* spp. cells. The variant Ascomycete fungal cells of any of numbered paragraphs 39-44, wherein the Ascomycete fungal cells are Hypocrea spp. cells.
46. The variant Ascomycete fungal cells of any of numbered paragraphs 39-45, wherein the Ascomycete fungal cells are *Trichoderma reesei* cells.

### BRIEF DESCRIPTION OF THE DRAWINGS

Figure 1 is a schematic diagram showing the features of a *tex1* disruption cassette.
Figure 2 is a schematic diagram showing the features of a *tex2* disruption cassette.
Figure 3 is a schematic diagram of genomic DNA from cells in which the *tex1* gene is disrupted using the described *tex1* disruption cassette, follow by selection for uridine auxotrophic mutants in which the *pyr2* gene introduced by the disruption cassette is deleted by recombination.

### DETAILED DESCRIPTION

### I. Overview

Without being limited to a theory, the described compositions and methods are based on the observation that the growth of Ascomycetes fungal cells on semi-solid substrates, including cellulosic "biomass" materials, may result in the production of peptaibols. These peptaibols can subsequently produce unwanted antimicrobial effects on the Ascomycetes fungal cells, themselves, or in subsequent processing steps, including those involving bacterial or yeast fermentation to produce ethanol or other valuable carbon-based products from biomass-derived sugars.

Peptaibols do not appear to be produced by Ascomycetes fungal cells in submerged (*i*.*e*., liquid) culture and are, therefore, not an issue for the production of industrially important polypeptides. However, substrates that contain significant amounts of relatively insoluble polymers, such as cellulose, lignin, xylan, and the like, present in the form of a slurry or solid suspension (herein collectively referred to as semi-solid suspension), represent growth medium conditions that resemble those of solid medium, as opposed to conventional submerged culture production medium. When Ascomycetes fungal cells are grown in such semi-solid medium, there may be increased probability of peptaibol production.

The compositions and methods relate to variant Ascomycetes fungal cells that are genetically-modified to reduce the production of peptaibols, particularly when grown on substrates containing insoluble materials. An exemplary use of the compositions and methods is the treatment of biomass substrates with live Ascomycetes fungal cells, or even cell-free compositions containing products from Ascomycetes fungal cells.

### II. Definitions

Prior to describing the present strains and methods in detail, the following terms arc defined for clarity. Terms not defined should be accorded their ordinary meanings as used in the relevant art.

The term "biomass" refers to cellulosic material (usually waste or other low value material) capable of being treated with digestive enzymes such as cellulases and hemicellulases to produce sugars, particularly six-carbon and five-carbon sugars. Exemplary components of biomass include but are not limited to wood chips, sawdust, corn stover, leaves, stems, branches of crop plants, and the like.

The term "Ascomycetes fungal cells" refers to any organism in the Division Ascomycota in the Kingdom Fungi. Exemplary Ascomycetes fungal cells include but are not limited to filamentous fungi in the subphylum Pezizomycotina, such as *Trichoderma* spp, *Aspergillis* spp, and *Penicillium* spp.

The term "*Trichoderma reesei"* refers to a filamentous fungus of the phylum Ascomycota, subphylum Pezizomycotina. This organism was previously classified as *Trichoderma longibrachiatum*, and also as *Hypocrea jecorina.*

The term "non-ribosomal peptide synthetase (NRPS)" refers to a non-ribosomal polypeptide capable of enzymatically linking amino (or imino) acid residues via peptide bonds. NRPS polypeptides are not associated with ribosomes or the canonical cellular translation process.

The term "peptaibols" refers to peptides of fungal origin having from about 7 to about 20 residues and featuring a high proportion of α-dialkylated amino acids (*e.g*., α-aminoisobutyric acid; Aib), *N-acyl* termini (usually Acetyl), and a C-terminal amino alcohol, such as phenylalaninol or leucinol. Peptaibols have broad spectrum antimicrobial activity against Gram-positive bacteria and fungi based on their ability to disrupt cell membranes.

The term "contact" means to bring into proximity as in the case of applying a solution or suspension of enzyme to a solid or semi-solid substrate or mixing a plurality of solutions or suspension such that molecules are allowed to interact.

The term "slurry," refers a mixture in which materials are partially-soluble in a given solvent, typically water. Examples of slurries include chemically-modified or gelatinized starch in water and modified *(e.g.,* chemically pretreated) cellulose in water. As used herein, at least 5% (w/v), at least 10%, at least 20%, at least 30%, at least 40%, or even at least 50% of total dry solid material in a slurry may be partially-soluble.

The term "solid suspension," refers a mixture in which at least a portion of dry solid materials are insoluble (and usually free-settling) in a given solvent, typically an aqueous solvent. As used herein, at least 5% (w/v), at least 10%, at least 20%, at least 30%, at least 40%, or even at least 50% of total dry solid material in a solid-suspension may be insoluble.

The term "semi-solid" or "semi-solid suspension" refers to a mixture of materials in which a significant amount (*e*.*g*., at least 5% (w/v), at least 10%, at least 20%, at least 30%, at least 40%, or even at least 50%) of partially-soluble and/or insoluble materials arc present. In some cases, the amounts of partially-soluble and/or insoluble materials may change over time (*e*.*g*., following enzymatic processing) but upon initial contact with Ascomycetes fungal cells, the mixture satisfies the definition of semi-solid suspension. Semi-solid suspensions include solid-suspensions and slurries.

The term "substantially aqueous solvent" refers to a solvent containing at least 50% (v/v) water, and preferably at least 60%, at least 70%, at least 80%, at least 90%, or even at least 95%, water, when first contacted with Ascomycetes fungal cells.

The term "at least partially insoluble" include materials that are insoluble (as in the case of solid suspension) and materials that are partially-soluble (as in the case of a slurry).

The term "fermenting" refers to the metabolism of sugar to produce a desired end product or intermediate, typically by bacteria or fungi.

The term "end product" refers to a desired chemical compound produced through the fermentation of sugars, in this case, sugars derived from biomass.

The term "intermediate" refers to a chemical compound produced through the fermentation of sugars, in this case, sugars derived from biomass, which chemical compound can be used to produce a desired end product.

The terms "polypeptide" and "protein" are used interchangeably to refer to polymers of any length comprising amino acid residues linked by peptide bonds. The conventional one-letter or three-letter codes for amino acid residues are used herein. The terms encompass amino acid polymers that have been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

The term "related proteins" refers to functionally and/or structurally similar proteins. Such proteins may be derived from organisms of different genera and/or species, or even different classes of organisms (*e*.*g*., bacteria and fungus). Related proteins also encompass homologs determined by primary sequence analysis, determined by secondary or tertiary structure analysis, or determined by immunological cross-reactivity.

The term "derivative polypeptide/protein" refers to a protein which is derived or derivable from a protein by addition of one or more amino acids to either or both the N- and C-terminal end(s), substitution of one or more amino acids at one or a number of different sites in the amino acid sequence, deletion of one or more amino acids at either or both ends of the protein or at one or more sites in the amino acid sequence, and/or insertion of one or more amino acids at one or more sites in the amino acid sequence. The preparation of a protein derivative may be achieved by modifying a DNA sequence which encodes for the native protein, transformation of that DNA sequence into a suitable host, and expression of the modified DNA sequence to form the derivative protein. Related (and derivative) proteins include "variant proteins." Variant proteins differ from a reference/parental protein (*e*.*g*., a wild-type protein) by substitutions, deletions, and/or insertions at small number of amino acid residues. The number of differing amino acid residues between the variant and parental protein may be one or more, for example, 1, 2, 3, 4, 5, 6, 7, 8, 9, 10, 15, 20, 30, 40, 50, or more amino acid residues. Variant proteins may share at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or even at least about 99%, or more, amino acid sequence identity with a reference protein. A variant protein may also differ from a reference protein in selected motifs, domains, epitopes, conserved regions, and the like.

The term "analogous sequence" refers to a sequence within a protein that provides similar function, tertiary structure, and/or conserved residues as the protein of interest (*i*.*e*., typically the original protein of interest). For example, in epitope regions that contain an α-helix or a β-sheet structure, the replacement amino acids in the analogous sequence preferably maintain the same specific structure. The term also refers to nucleotide sequences, as well as amino acid sequences. In some embodiments, analogous sequences are developed such that the replacement amino acids result in a variant enzyme showing a similar or improved function. In some embodiments, the tertiary structure and/or conserved residues of the amino acids in the protein of interest are located at or near the segment or fragment of interest. Thus, where the segment or fragment of interest contains, for example, an α-helix or a β-sheet structure, the replacement amino acids preferably maintain that specific structure.

The term "homologous protein" refers to a protein that has similar activity and/or structure to a reference protein. It is not intended that homologs necessarily be evolutionarily related. Thus, it is intended that the term encompass the same, similar, or corresponding enzyme(s) *(i.e.,* in terms of structure and function) obtained from different organisms. In some embodiments, it is desirable to identify a homolog that has a quaternary, tertiary and/or primary structure similar to the reference protein. In some embodiments, homologous proteins induce similar immunological response(s) as a reference protein. In some embodiments, homologous proteins are engineered to produce enzymes with desired activity(ies).

The term "gene" is synonymous with the term "allele" in referring to a nucleic acid that encodes and directs the expression of a protein or RNA. Vegetative forms of Ascomycetes fungal cells are generally haploid, therefore a single copy of a specified gene (*i.e.,* a single allele) is sufficient to confer a specified phenotype.

The terms "wild-type," "native," or "endogenous" with reference to genes and proteins refers to those found in nature.

The term "disruption of a gene" refers broadly to any genetic manipulation that substantially prevents a cell from producing a function gene product, *e*.*g*., a protein encoded by the gene, in a host cell. Exemplary methods of disruption include complete or partial deletion of any portion of a gene, including a polypeptide-coding sequence, a promoter, an enhancer, or another regulatory element, or mutagenesis of the same, where mutagenesis encompasses substitutions, insertions, deletions, inversions, and combinations and variations, thereof, any of which mutations substantially prevent the production of a function gene product.

The term "deletion of a gene" refers to its removal from the genome of a host cell. Where a gene includes control elements (*e*.*g*., enhancer elements) that are not located immediately adjacent to the coding sequence of a gene, deletion of a gene refers to the deletion of the coding sequence, and optionally adjacent promoter and/or terminator sequences.

The term "genetic modification" refers to the modification of a preselected nucleic acid target sequence, *e.g.,* using macromolecules *(i.e.,* enzymes and/or nucleic acids) that preferentially act on the preselected nucleic acid sequence. In this manner genetic manipulation is distinct from chemical manipulation, in which small molecules are used to randomly affect changes to a nucleic acid sequence that is not preselected.

The term "fermenting microorganism" refers to any microorganism suitable for use in a desired fermentation process. Suitable fermenting microorganisms according to the instant disclosure are able to ferment, *i.e.,* convert, sugars, such as, for example, glucose, xylose, arabinose, mannose, galactose, or oligosaccharides, directly or indirectly into a desired end product or intermediate.

The term "aerobic fermentation" refers to growth in the presence of oxygen.

The term "functional polypeptide/protein" is a protein that posses an activity, such as an enzymatic activity, a binding activity, a surface-active property, or the like, and which has not been mutagenized, truncated, or otherwise modified to abolish or reduce that activity. Functional polypeptides may be thermostable or thermolabile, as specified.

The term "functional gene" refers to a gene capable of being used by cellular components to produce an active gene product, typically a protein. Functional genes are distinguished from disrupted genes, which are modified such that they cannot be used by cellular components to produce an active gene product.

A "protein of interest" is a protein that is desired to be produced in Ascomycetes fungal cells. Generally, proteins of interest are commercially important for industrial or pharmaceutical use, making them desirable to produce in large quantities. In some cases, proteins of interest hydrolyze biomass substrates.

A genetic modification does not "significantly reduce the production or secretion of a protein of interest" if the difference in protein of interest expression between cells having the genetic modification and cells lacking the genetic modification is less than about 20%, less than about 15%, less than about 10%, less than about 7%, less than about 5%, or even less than about 3%.

The singular articles "a," "an," and "the" encompass the plural referents unless the context clearly dictates otherwise. Where used in the description, the following abbreviations/acronyms have the following meanings unless otherwise specified:
- EC: enzyme commission
- kDa: kiloDalton
- kb: kilobasc
- MW: molecular weight
- w/v: weight/volume
- w/w: weight/weight
- v/v: volume/volume
- wt%: weight percent
- °C: degrees Centigrade
- H₂O: water
- H₂O₂: hydrogen peroxide
- dH₂O or DI: deionized water
- dIH₂O: deionized water, Milli-Q filtration
- g or gm: gram
- µg: microgram
- mg: milligram
- kg: kilogram
- lb: pound
- µL and µl: microliter
- mL and ml: milliliter
- mm: millimeter
- µm: micrometer
- M: molar
- mM: millimolar
- µM: micromolar
- U: unit
- ppm: parts per million
- sec and": second
- min and': minute
- hr: hour
- EtOH: ethanol
- eq.: equivalent
- N: normal
- PCR: polymerase chain reaction
- DNA: deoxyribonucleic acid
- FOA: fluoroorotic acid
- UV: ultraviolet
- A₅₄₀: absorbance measured at a wavelength of 540 nm
- CMC: carboxymethyl cellulose
- rpm: revolutions per minute
- Δ: relating to a deletion
- DO: dissolved oxygen
- MALDI-TOF: matrix-assisted laser desorption/ionization time of flight mass spectrometry
- TFA: trifluoroacetic acid

### III. Preferred Embodiments

The present compositions and methods are based, in part, on the theory that the growth of Ascomycetes fungal cells on semi-solid substrates, such as cellulosic "biomass" materials, results in the production of peptaibols, which are known to be produced on solid medium but not in submerged culture. These peptaibols can produce unwanted antimicrobial effects on the Ascomycetes fungal cells, themselves, and/or in subsequent substrate processing steps, particularly those involving bacterial or yeast fermentation to produce ethanol or other valuable carbon-based products from sugars produced by the Ascomycetes fungal cells.

Peptaibols are linear peptides produced in a variety of Ascomycetes fungal cells by non-ribosomal peptide synthetases (NRPSs). Peptaibols are typically no more than about 20 residues in length, with C-terminal alcohol residues, non-standard amino acid residues, including α-aminoisobutyric acid (Aib) and isovaline (Iva), and the imino acid, hydroxyproline (Hyp). Peptaibols have broad spectrum antimicrobial activity against bacteria and yeast, including Ascomycetes fungal cells, because of their ability to form ion channels in membranes.

For the purposes of the present methods, suitable examples of Ascomycetes fungal cells that produce peptaibols include, but are not limited to, *Acremonium* spp., *Apiocrea* spp., *Boletus* spp., *Cephalosporium* spp., *Clonostachys* spp., *Emericellopsis* spp., *Gliocladium* spp., *Hypocrea* spp., *Mycogone* spp., *Sepedonium* spp., *Silbella flavipes* spp., *Stilbella* spp., *Tolypocladium* spp., *Trichoderma* spp., *Tylopilus* spp., and *Verticimonosporium* spp. *Trichoderma* spp., in particular, are known to produce significant amount of peptaibols on solid medium. *Trichoderma* spp. include *T. atroviride, T.flavofuscum, T. ghanense, T. harzianum, T. harzianun, T*. *koningii, T. longibrachiatum, T. parareesei, T. polysporum,* T. *pseudokoningii, T. reesei, T. saturnisporum,* and *T. viride.* The sequence of individual peptaibols from such species can be found, for example, in the online Peptaibol Database maintained by the School of Crystallography, Birkbeck College, Univ. of London, United Kingdom.

NRPSs are modular polypeptides produced by Ascomycetes fungal cells. Each module includes an adenylation (A) domain, a thiolation (T) domain, and a condensation (C) domain), which together catalyze the incorporation of a single amino acid residue into a peptaibol molecule. The number of modules in an NRPS determines the maximum number of residues in the peptaibols produced. For example, an 18-module NRPS generates an 18-residue peptaibol, and so forth. However, a single NRPS can produce shorter peptaibols through module skipping. NRPS have largely been characterized in *Trichoderma* spp., although they have been identified in a variety of other Genera, for example, *Penicillium.* A Database of NonRibosomal Peptide Synthetases is maintained by the National Institute of Immunology, New Delhi, India. One previously described model NRPS is tex1 in *Trichoderma* spp., which is encoded by a 62.8-kb *tex1* gene. Disruption of the *tex1* gene prevents the synthesis of all 18-residue peptaibols, and would be expected to prevent the synthesis of shorter peptaibols that result from module skipping.

According to the present methods, variant Ascomycetes fungal cells are provided, which include a genetic modification that reduces the production of at least one peptaibol, or at least one family of peptaibols. In some embodiments, the genetic modification is the disruption of a gene controlling the production of a peptaibol, such as an NRPS. Disruptions of NRPS genes include genetic manipulations that substantially prevent a cell from producing a function NPRS gene product, *i.e.,* a functional NPRS protein.

Exemplary disruptions include the complete or partial deletion of any portion of a gene, including a polypeptide-coding sequence, a promoter, an enhancer, or another regulatory element, or mutagenesis of the same, where mutagenesis encompasses substitutions, insertions, deletions, inversions, and combinations and variations, thereof, any of which mutations substantially prevent the production of a function gene product. In view of the unusually large size and repetitive modular nature of NPRS genes, a preferred method of disrupting an NPRS gene involves mutating or deleting at least a portion of the promoter.

In some embodiments, a single NPRS is disrupted, ideally the NPRS responsible for the production of the peptaibol, or class or peptaibols, that is most antimicrobial to the particular Ascomycetes sp. fungal cells, themselves, or to bacteria and/or fungi involved in subsequent processing steps. In some embodiments, multiple NPRS are disrupted. NPRS can be disrupted using standard molecular biology methods, typically involving site specific recombination using a deletion cassette, as exemplified in the appended Examples. In some embodiments, NPRS genes are disrupted without introducing foreign DNA into the variant cells, *i.e.,* without introducing DNA derived from other microbes or vector DNA.

The resulting genetically-modified Ascomycetes sp. fungal cells produce reduced amounts of one or more peptaibols compared to the otherwise equivalent parental Ascomycetes fungal cells. Methods for the detection and quantitation of peptaibols are well-known in the art and primarily involve matrix-assisted laser desorption/ionization time of flight mass spectrometry (MALDI-TOF) analysis. In some embodiments, the variant genetically modified Ascomycetes sp. fungal cells produce less that 50%, less that 60%, less that 70%, less that 80%, less that 90%, or even less that 95% of the amount of particular unwanted peptaibols, or class of peptaibols compared to the otherwise equivalent parental Ascomycetes fungal cells. In some embodiments, the genetically modified Ascomycetes fungal cells do not produce any of such peptaibols.

The parental Ascomycetes fungal cells may be wild-type, or may have any number of genetic modifications other than those specifically made to alter peptaibol production. Typical genetic modifications to the parental Ascomycetes fungal cells include the introduction of a gene encoding a protein of interest (particularly enzymes for digesting biomass), the disruption of genes encoding unwanted proteins (such as proteases), the introduction of selectable markers, integration cassettes, or other features to facilitate further genetic manipulation, and the introduction of mutations that affect cell morphology, protein production, or secretion.

Where the reduction of peptaibols in the variant genetically modified Ascomycetes fungal cells results in improved growth of variant Ascomycetes fungal cells, themselves, the improved growth may be in the form of increased growth rate, prolonged growth, increased or prolonged production of a proteins of interest, or combinations, thereof.

In some embodiments, the Ascomycetes fungal cells produce and secrete enzymes that hydrolyze biomass substrates. Such enzymes include, but are not limited to, cellulases, hemicellulases, and esterases. Such enzymes may be endogenous or exogenous. In some cases, The Ascomycetes fungal cells produce a number of such enzymes in specific ratios. A feature of the present compositions and methods is that the genetic modification that reduces the production of peptaibols does not significantly reduce the production or secretion of the enzymes the hydrolyze biomass.

Although the present composition are not limited to particular Ascomycetes fungal cells, exemplary Ascomycetes fungal cells are filamentous fungi in the subphylum Pezizomycotina, including commercially important production host cells such as *Trichoderma*, including *T*. *reesei,* which are excellent production hosts and are known to produce significant levels of peptaibols when grown on solid medium.

The present methods are further not limited to a particular type of semi-solid suspension of material, although a representative material is biomass. Generally, the semi-solid material has at least one component which is at least partially insoluble, which includes materials that are insoluble (as in the case of solid suspension) and materials that are partially-soluble (as in the case of a slurry). In some embodiments, at least 5% (w/v), at least 10%, at least 20%, at least 30%, at least 40%, or even at least 50% of the total dry weight of the substrate is either partially-soluble or insoluble. The amounts of partially-soluble and insoluble materials may change over time, *e*.*g*., following enzymatic modification and/or processing of large polymers, and/or following the production of an end product or intermediate. Accordingly, some of the partially-soluble and insoluble material may become soluble due to the action of enzymes produced by the variant genetically-modified Ascomycetes fungal cells, while some material may remain partially-soluble or insoluble and eventually be discarded as waste material.

Non-limiting examples of components of biomass include wood waste (*e*.*g*., chips, sawdust), plant materials left over from crop harvests (*e.g*., corn stover, leaves, stems, and branches), recycled paper, and the like. Biomass may include cellulose, hemicellulose (*e*.*g*., xylan), lignin, resins, and other naturally-occurring plant materials, and may be pretreated prior to being contacted with Ascomycetes fungal cells.

Semi-solid suspensions of substrates, including but not limited to biomass, may be contacted with the variant Ascomycetes fungal cells producing enzymes for the hydrolysis of the substrate into sugars (*e*.*g*., glucose, xylose, arabinose, mannose, galactose, or oligosaccharides), which are fermentable by a second, fermenting organism. Cells of the fermenting organism may be present at the same time as the variant Ascomycetes fungal cells (*i*.*e*., simultaneously), may be contacted with the hydrolyzed substrate following contact with the variant Ascomycetes fungal cells *(i.e.,* sequentially), or may be contacted with the hydrolyzed substrate following contact with a protein of interest produced by the variant Ascomycetes fungal cells. In some cases, the pH, salt concentration, temperature, or other conditions may be adjusted to better suit the cells of the second, fermenting organism.

The second, fermenting organism may produce a desired end product, or an intermediate, thereof. End products and intermediates include, for example methanol, ethanol, butanol, acetone, acetic acid, methane, ethane, and a various aldehydes, ketones, and phenolic compounds. The fermenting organism may be bacterial, fungal, or a plurality or combination, thereof. Because peptaibols are known to have broad spectrum antimicrobial effects on both bacteria and fungi, the present compositions and methods are expected to promote the growth and/or productivity of a variety of organisms capable of fermenting the sugars produced by the Ascomycetes fungal cells.

Examples of suitable fermenting microorganisms include, without limitation, fungal organisms, such as yeast. Exemplary yeast are *Saccharomyces* spp., *e.g., S. cerevisiae, S. distaticus*, or *uvarum*, *Kluyveromyces* spp., *e.g., K. marxianus* and *K. fragilis, Candida* spp., *e.g., C. pseudotropicalis* and C. *brassicae, Clavispora* spp. *e.g., C. lusitaniae* and C. *opuntiae, Pachysolen* spp., *e*.*g*., *P. tannophilus*, *Bretannomyces* spp., *e*.*g*., *B. clausenii,* and commercially available yeast such as ETHANOL RED™ yeast (available from Fermentis/Lesaffre, USA), FALI (available from Fleischmann's Yeast, USA), SUPERSTART™ and THERMOSACC® fresh yeast (available from Ethanol Technology, WI, USA), BIOFERM AFT and XR (available from NABC-North American Bioproducts Corporation, GA, USA), GERT STRAND (available from Gert Strand AB, Sweden), or FERMIOL (available from DSM Specialties). Yeast fermentation has been described in the literature *(see, e.g.,* Philippidis (1996) Cellulose bioconversion technology, in Handbook on Bioethanol: Production and Utilization, (Wyman, ed., Taylor & Francis, Washington, D.C., 179-212). Bacteria that can efficiently ferment glucose to ethanol include, for example, *Zymomonas mobilis* and *Clostridium thermocellum (see, e,g.,* Philippidis (1996) *supra).*

The expression of heterologous genes in *Saccharomyces cerevisiae (see, e.g.,* Chen and Ho (1993) Appl. Biochem. Biotechnol. 39-40:135-147; Ho et al. (1998) Appl. Environ. Microbiol. 64:1852-1859), *Escherichia coli* (*see, e*.*g*., Beall et al. (1991) Biotech. Bioeng. 38: 296-303), *Klebsiella oxytoca* spp. (*see, e.g.,* Ingram, et al. (1998) Biotechnol. Bioeng. 58:204-214), and *Zymomonas mobilis* (*see, e.g.,* Zhang et al. (1995) Science 267:240-243; Deanda et al. (1996) Appl. Environ. Microbiol. 62:4465-4470) has further led to the engineering of organisms capable of converting hexoses and pentoses to ethanol (cofermentation), which are particularly valuable for converting sugars generated from biomass substrates in to end products and intermediates. Particular hexose/pentose-fermenting organisms include the variant *Zymomonas mobilis* spp. cells described in, *e.g.,* U.S. Patent Publication Nos. US 2009/0221078, US 2009/0246846, and US 2003/0162271.

Effects of reduced peptaibol production by the Ascomycetes fungal cells on fermenting organisms include increased growth rate *(i.e.,* faster growth and/or prolonged growth), improved yield of end products or intermediates, or a combination, thereof. Such improvements can be measured relative to the growth of the same second organisms in partially hydrolyzed biomass substrate obtained using Ascomycetes fungal cells that have not been genetically modified to reduce the production of peptaibols. The improvement in any of the aforementioned growth parameter may be at least 10%, at least 20%, at least 30%, at least 40%, at least 50%, at least 60%, at least 70%, at least 80%, at least 90%, at least 100%, or more.

### EXAMPLES

### Example 1. Disruption of the tex1 and tex2 genes from a T. reesei strain to obtain variants with reduced peptaibol production

### 1.1. Media and stock solutions

Media and other stock solutions used in the Examples arc described, below:

| VOGEL'S MINIMAL MEDIUM (VM) 1 L | |
|---|---|
| 50x Vogel's Solution | 20 mL |
| 50% Glucose | 20 mL |
| Add dH₂O to final volume | |
| Agar | 20 g |
| Autoclave | |

| 50x VOGELS SOLUTION | |
|---|---|
| Sodium Citrate.2H₂O | 125 g |
| KH₂PO₄ (Anhydrous) | 250 g |
| NH₄NO₃ | 100 g |
| MgSO₄.7H₂O | 10 g |
| CaCl₂.2H₂O | 5 g |
| Vogel's Trace Element Solution | 5 mL |
| Vogel's Biotin Solution | 2.5 mL |
| Add dH₂O to final volume | |
| Filter sterilize | |

| VOGEL'S TRACE ELEMENT SOLUTION | |
|---|---|
| Citric Acid | 50 g |
| ZnSO₄.7H₂O | 50 g |
| Fe(NH₄)₂SO₄.6H₂O | 10 g |
| CuSO₄.5H₂O | 2.5 g |
| MnSO₄.4H₂O | 0.5 g |
| H₃BO₃ | 0.5 g |
| Na₂MoO₄.2H₂O | 0.5 g |
| Add dH₂O to final volume | |
| Filter sterilize | |

| VOGEL'S BIOTIN SOLUTION 1 L | |
|---|---|
| D-Biotin | 0.1 g |
| Filter sterilize | |

| POTATO DEXTROSE AGAR (PDA) 1 L | |
|---|---|
| BD Difco Potato Dextrose Agar | 39 g |
| Add dH₂O to final volume | |
| Autoclave | |

| *T. reesei* GLYCINE MINIMAL MEDIUM 1 L | |
|---|---|
| Glycine | 6 g |
| (NH₄)₂SO₄ | 4.7 g |
| KH₂PO₄ | 5 g |
| MgSO₄.7H₂O | 1 g |
| 100mg/mL CaCla*2H₂O solution | 10 mL |
| *T.reesei* Trace Elements 400X | 2.5 mL |
| PIPPS | 33 g |
| Adjust pH to 5.50 with 50% NaOH | (about 5 mL per liter). |
| Add dH₂O to 950 ml | |
| Autoclave. | |
| Add 40% Lactose | 50 mL |

| *T*. *reesei* TRACE ELEMENTS 400x 1 L | |
|---|---|
| Citric Acid (Anhydrous) | 175 g |
| FeSO₄.7H2O | 200 g |
| ZnSO₄.7H2O | 16 g |
| CuSO₄.5H2O | 3.2 g |
| MnSO₄.H2O | 1.4 g |
| H₃BO₃ (Boric Acid) | 0.8 g |

### 1.2. Generation of a tex1 disruption cassette

A disruption cassette is generated to delete the 5' end of the *tex1* gene in *T*. *reesei.* Disruption of the 5' portion of the gene is sufficient to abolish expression and avoids deleting the entire *tex1* gene, which is substantial in size. To assemble a DNA construct designed to delete the 5' end of the *tex1* gene two DNA fragments are amplified by PCR. Firstly, an approximately 1-kb region of genomic DNA upstream of the *tex1* coding region (called the 5' region) is amplified by PCR using genomic DNA from *T*. *reesei* strain QM6a as a template. Two primers are designed for this PCR, one primer (the forward primer) has a *Not*I recognition site close to the 5' end and hybridizes to the genomic DNA at a position approximately 1.5 kb upstream (5') of the start codon of the *tex1* gene and the second primer (the reverse primer) has an *Mfe*I recognition site close to the 5' end and hybridizes to the genomic DNA at a position approximately 0.5kb upstream (5') of the start codon of the *tex1* gene. Second, an approximately 1-kb region of genomic DNA within the coding region of the *tex1* gene (called the 3' region) is amplified by PCR using genomic DNA from *T. reesei* strain QM6a as a template. Two primers are designed for this PCR, one primer (the forward primer) has an *Mfe*I recognition site close to the 5' end and hybridizes to the genomic DNA at a position approximately 1 kb downstream (3') of the start codon of the *tex1* gene and the second primer (the reverse primer) has a *Not*I recognition site close to the 5' end and hybridizes to the genomic DNA at a position approximately 2 kb downstream (3') of the start codon of the *tex1* gene. Each PCR product is digested with *Mfe*I and *Not*I*.* These fragments of DNA are ligated with pZErO-2 vector (Invitrogen Corporation, Carlsbad, CA) vector that has been digested with *Not*I and introduced into *E. coli* cells by transformation. After propagation in *E*. *coli,* the assembled vector with inserted PCR products is isolated, digested with *Mfe*I and treated with alkaline phosphatase to dephosphorylate the DNA ends.

A third DNA fragment of approximately 0.5 kb from further downstream within the *tex1* coding region is amplified (called the 3' repeat). Two primers are designed for this PCR, one primer (the forward primer) has an *Eco*RI recognition site close to the 5' end and hybridizes to the genomic DNA at a position approximately 2.5 kb downstream (3') of the start codon of the *tex1* gene and the second primer (the reverse primer) has an *Mfe*I recognition site close to the 5' end and hybridizes to the genomic DNA at a position approximately 2.5 kb downstream (3') of the start codon of the *tex1* gene. This PCR product is digested with *Eco*RI and *Mfe*I*,* and ligated with the *Mfe*I-digested and dephosphorylated vector DNA from above. Following propagation in *E. coli,* restriction digestion of the plasmids created by this ligation identifies clones in which the 0.5 kb PCR product is present in the desired orientation. The assembled vector with three inserted PCR products is digested with *Mfe*I and treated with alkaline phosphatase to dephosphorylate the DNA ends.

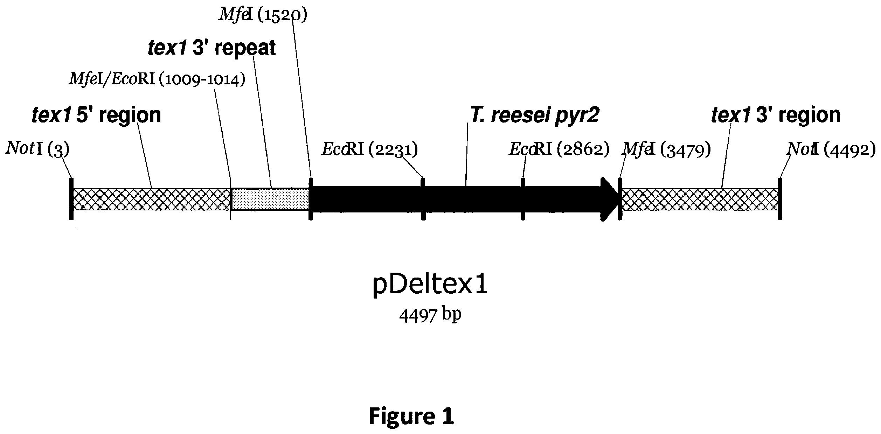

A DNA fragment that includes the functional promoter, coding and terminator regions of the *T. reesei pyr2* gene (encoding orotate phosphoribosyltransferase) is amplified by PCR using genomic DNA from *T*. *reesei* strain QM6a as a template. The forward and reverse primers used for this amplification each have *Mfe*I recognition sites close to the 5' end. This PCR product is digested with *Mfe*I and ligated with the *Mfe*I-digested and dephosphorylated vector DNA from above. The final vector is called pDeltex1. The *tex1* disruption cassette comprising the *tex1* 5' region, the *T*. *reesei pyr2* gene, the *tex1* 3' region and the *tex1* 3' repeat is isolated from this vector by digestion with *Not*I or by PCR amplification (Figure 1). The nucleotide sequence of this disruption cassette is given as SEQ ID NO: 1. Table 1 shows the individual regions within SEQ ID NO: 1 by nucleotide number.

**Table 1. Features of an exemplary tex1 disruption cassette**

| Nucleotide position | Function |
|---|---|
| 1-8 | *Not*I recognition site |
| 9-1008 | *tex1 5'* region |
| 1009-1014 | Hybrid *Mfe*I*lEcoR*I site |
| 1015-1518 | *tex1* 3' repeat |
| 1519-1524 | *Mfe*I recognition site |
| 1525-3477 | *T*. *reesei pyr2* gene (including promoter and terminator) |
| 3478-3483 | *Mfe*I recognition site |
| 3484-4489 | *tex1* 3' region |
| 4490-4497 | *Not*I recognition site |

SEQ ID NO: 1:

gcggccgcgtgcgaaccgactctactgtcaacctcgaaacatacaattaccagagcttttcagactcgacagacggaacatgtagagtttc
gaatcccgtgagtggctgcaatgatgtatttgttgtgttgccggttgtggtcttgcgaaaactgcagggtagcctttactcgatgaaagtccga
cagaggcgccttgactgagccaatcagttgctcaacgtcaaccgatgtgtcaatggaatgtggatatgtacttctgtcgtccagtcagacga
gagtcgcttcttcccatgttggagccttgcctccagagtcctcaccgaagtacgagtacctgctcattgactaggtaccttaggtatgtgctcta
cctaggcgccgaaccctctctacttctccggagccttgctgtttggcgcccgccaagcaacacacctgcatagacatccctgtccagtttga
gtcgtctccagttttctctccccggcttcatctccagctggcccgggggaccctgtaagaccctgtccctgagaggcaaagctgccatcct
gcttcctgggctgctcatcagtgctgctaaagctgacttgttattcaccgatacaggtcgaatgctgactacgaacgcctctttctcgcatacat
tggtcctgaacattgtctaagacaaccaccccccttgccgcggcacagctgaccatcagcctagagccagcctcccgtctgtgggatctg
tgagggatcctgcaatgggctgatcctggttctgcttctggactgaaccccattgagtctcacgcggaagaaagagtgtgctacctcggcca
ggcctcaaacactgctctgtagctgtaatacgactgctgggtattattattgatggccgggaggaaccgagccagggctgcacagtgtcga
cggaggtacctcctcgtgggagcagcgtatccgctacggataccccagcatatgcatcaacctcgtaataggtagcgactgatcaattcctg
ctgttggaaggctgtggcaggagtcggctgtgccgaatctcgcccctttgttctcccacggcagggcaatttctgggttacctcagtatccct
ggagttacggaacctctgatgaggactccgatgctactgctctatctgcccgtgagaagcgggctcgtgtctttgcagaggacagctctctg
gagagcgagctcgccgacgaagaggctcctaccactgaggtggagattgttcttcgagctgtctgggttgagatcctccgggatgtgaacc
gaatccgtaagcaggacattggaaagaagcacaacttcttcctgcttggcggcgactctctcacaaccatcgagcttgttactgctgcttatc
aatacggcattcgtctgagtccggcagccgtatcggacaacgctgagcttgcccagatggcagctgtggcaaccatcgaacacgacagc
gcaatgatggctgagacaaagcccttcagcttgattagcagtgcaattggctagctagttacgcttgtttatttacgacaagatctagaagattc
gagatagaataataataataacaacaatttgcctcttctttccaccttttcagtcttactctcccttctgacattgaacgcctcaatcagtcagtcgc
cttgtacttggcacggtaatcctccgtgttcttgatatcctcaggggtagcaaagcccttcatgccatcgataatgtcatccagagtgaggatg

gcaaagatggggatgccgtactccttcctcagctcgccaatggcactcggtccaggcttggagtcgtcgccatccgcagcggggagcttct
ccatgcggtccagggccacgacgatgccggcgacgatgccgccctccttggtgatcttctcaatggcgtccctcttggcggtgccggcgg
tgatgacgtcgtcgacaatcaggaccctcttgcccttgagcgaagcgccgacgatgttgccgccctcgccgtggtccttggcctccttgcg
gtcaaacgagtaggagacgcggtccaggttctggggcgccagctcgccgagcttgatggtgatggcggagcacagcgggatgccccttgt
aggccgggccgaagacgatgtcgaactctaggccggccttctcctgggcctcgatgatggtctttgcaaaggcggaggcgatggcgccg
gcgaggcgcgccgtgtggaattcgcccgcgttgaagaagtaggggggatatccgcttggacttgagctcgaagctgccaaacttgaggac
gccgccgtcgatggcggatttgaggaagtcctgcttgtaggcaggcagctgggaggtggtagccattctgttggatttggatagtgtccttat
tctctgatttgaacagtagatcaggacgagtgagagggatgcagaggttggattggagtggttgagctataaaatttagaggcgcgccgtat
cgagttttcacatggaagtcaaagcgtacagtgcgagcttgtacgttggtcttagtatcccacaagcttctgtctaggtatgatgatggctataa
gtcacccaaggcagaactcatcttgaagattgtctagagtgattttaccgctgatgaaatgactggactccctcctcctgctcttatacgaaaa
attgcctgactctgcaaaggttgtttgtcttggaagatgatgtgccccccatcgctcttatctcataccccgccatctttctagattctcatcttca
acaagaggggcaatccatgatctgcgatccagatgtgcttctggcctcatactctgccttcaggttgatgttcacttaattggtgacgaattcag
ctgatttgctgcagtatgctttgtgttggttctttccaggcttgtgccagccatgagcgctttgagagcatgttgtcacctataaactcgagtaac
ggccacatattgttcactacttgaatcacatacctaattttgatagaattgacatgtttaaagagctgaggtagctttaatgcctctgaagtattgt
gacacagcttctcacagagtgagaatgaaaagttggactccccctaatgaagtaaaagtttcgtctctgaacggtgaagagcatagatccgg
catcaactacctggctagactacgacgtcaattctgcggccttttgacctttatatatgtccattaatgcaatagattcttttttttttttttttttttt
ttttttttttttttgcccaatttcgcagatcaaagtggacgttatagcatcataactaagctcagttgctgagggaagccgtctactaccttagccc
atccatccagctccataccttgatactttagacgtgaagcaattcacactgtacgtctcgcagctctccttcccgctcttgcttccccactgggg
tccatggtgcgtgtatcgtcccctcccaattgcgtggtaacattggagaaccggacaatctctccgaatattgtatctggtaagtttcatgctgc
tcaagtccgccgaaaagtcaggcccaagtctctaacgtaaagaaacatcagcatcctctacccccggagcgctcgtagctacgcaggcca
ttccgttcccgcatgatcgcgcagagagagccagcatcattgcctctgacgctgagggacacagcgctcatgttatcgtcgactctcacccc
cggcctactccgcgagtatctgagtcccggccgcaacttgttctgttctctgcgaacaacccagcttctttggctaagcagatcgagctgcac
cgcctgtatgctgaatctcacccagacgatgctgccgacatcgcctatactcgcgctctccgacgcgaggctctggactacaaggcattctc
catcctttcgaactcatccttcgtcaacacttccaactacgccaagtcttccgcgcgggctccggccatcaccatggtgttcaacggccagg
gtgctcagtgggcgggaatgggcaaggagctcatcttgaccgactcaagcttcagggaagacatcaggaagatggatctggttctgaagg
gactgaacatacccgcaacctggtcgattgaagaggagcttctccgtgatgctgccgaccccgacaacaggatcaacacctcaaagttctc
ctaccctctcagcacggcgcttcagattgccctggtcaactgtttcaaacgccttggtgttacgcccaaggccgtcgttggtcactccagtgg
tgagattgctgctgcctatgctgcgggctttctctctttcgaagacgccatcacagtcgcttactactacggccatattactaccagggaccag
aaggacggtgccatgggcgtggtaagcttaggtgcagaggaaacggaggggtttctcgagcagggcgtggtcattgcttgtgagaactc
acccactagcaccaccatttccggcgagcggccgc

### 1.3. Generation of a tex2 disruption cassette

A disruption cassette is generated to delete the 5' end of the *tex2* gene in *T. reesei.* Disruption of the 5' portion of the gene is sufficient to abolish expression and avoids deleting the entire *tex2* gene, which is substantial in size. In order to assemble a DNA construct designed to delete the 5' end of the *tex2* gene DNA fragments are amplified by PCR. First, an approximately 1-kb region of genomic DNA upstream of the *tex2* gene (called the 5' region) is amplified by PCR using genomic DNA from *T*. *reesei* strain QM6a as a template. Two primers are designed for this PCR, one primer (the forward primer) has a *Not*I recognition site close to the 5' end and hybridizes to the genomic DNA at a position approximately 1.5 kb upstream (5') of the start codon of the *tex2* gene and the second primer (the reverse primer) has an *Xma*I recognition site close to the 5' end and hybridizes to the genomic DNA at a position approximately 0.5 kb upstream (5') of the start codon of the *tex2* gene. Second, an approximately 1-kb region of genomic DNA within the coding region of the *tex2* gene (called the 3' region) is amplified by PCR using genomic DNA from *T*. *reesei* strain QM6a as a template. Two primers are designed for this PCR, one primer (the forward primer) has an *Xma*I recognition site close to the 5' end and hybridizes to the genomic DNA at a position approximately 1 kb downstream (3') of the start codon of the *tex2* gene and the second primer (the reverse primer) has a *Not*I recognition site close to the 5' end and hybridizes to the genomic DNA at a position approximately 2 kb downstream (3') of the start codon of the *tex2* gene. Each PCR product is digested with *Xma*I and *Not*I*.* These fragments of DNA arc ligated with pZErO-2 vector (Invitrogen Corporation, Carlsbad, CA) that has been digested with *Not*I and introduced into *E*. *coli* cells by transformation. After propagation of *E*. *coli*, the assembled vector with inserted PCR products is isolated, digested with *Xma*I and treated with alkaline phosphatase to dephosphorylate the DNA ends.

A third DNA fragment of approximately 0.5 kb from further downstream within the *tex2* coding region (called the 3' repeat) is amplified. Two primers are designed for this PCR, one primer (the forward primer) has an *Ngo*MIV recognition site close to the 5' end and hybridizes to the genomic DNA at a position approximately 2.5 kb downstream (3') of the start codon of the *tex2* gene and the second primer (the reverse primer) has an *Xma*I recognition site close to the 5' end and hybridizes to the genomic DNA at a position approximately 2.5 kb downstream (3') of the start codon of the *tex2* gene. This PCR product is digested with *Ngo*MIV and *Xma*I*,* and ligated with the *Xma*I-digested and dephosphorylated vector DNA from above. Following propagation in *E*. *coli,* restriction digestion of the plasmids created by this ligation identifies clones in which the 0.5-kb PCR product is present in the desired orientation. The assembled vector with three inserted PCR products is digested with *Xma*I and treated with alkaline phosphatase to dephosphorylate the DNA ends.

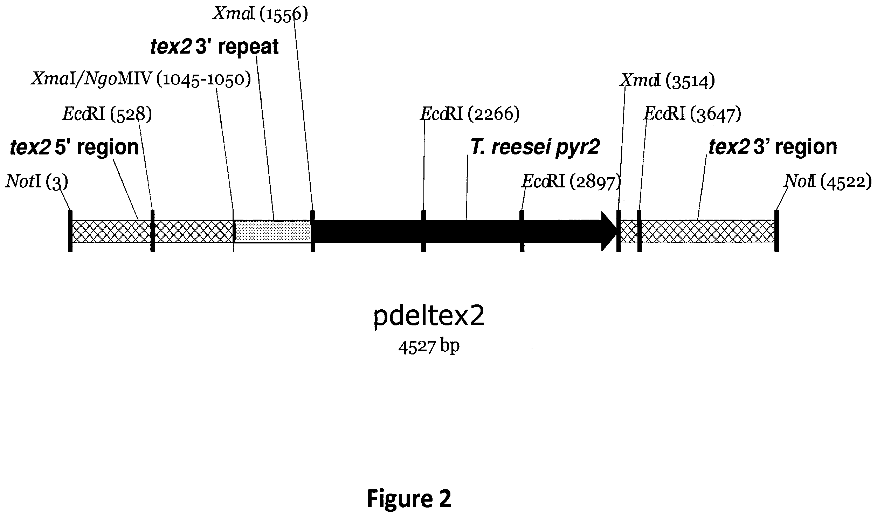

A DNA fragment that includes the functional promoter, coding and terminator regions of the *T. reesei pyr2* gene (encoding orotate phosphoribosyltransferase) is amplified by PCR using genomic DNA from *T. reesei* strain QM6a as a template. The forward and reverse primers used for this amplification each have *Xma*I recognition sites close to the 5' end. This PCR product is digested with *Xma*I and ligated with the *Xma*I-digested and dephosphorylated vector DNA from above. The final vector is called pDeltex2. The *tex2* disruption cassette comprising the *tex2* 5' region, the *T. reesei pyr2* gene, the *tex2* 3' region and the *tex2* 3' repeat is isolated from this vector by digestion with *Not*I or by PCR amplification (Figure 2). The nucleotide sequence of this disruption cassette is given as SEQ ID NO: 2. Table 2 shows the individual regions within SEQ ID NO: 2 by nucleotide number.

**Table 2. Features of an exemplary tex1 disruption cassette**

| Nucleotide position | Function |
|---|---|
| 1-8 | *Not*I recognition site |
| 9-1044 | *tex2* 5' region |
| 1045-1050 | Hybrid *Xma*I/*Ngo*MIV site |
| 1051-1554 | *tex2* 3' repeat |
| 1555-1560 | *Xma*I recognition site |
| 1561-3512 | *T. reesei pyr2* gene (including promoter and terminator) |
| 3513-3518 | *Xma*I recognition site |
| 3519-4519 | *tex2* 3' region |
| 4520-4527 | *Not*I recognition site |

SEQ ID NO: 2:

gcggccgcgagttcacatcagctccttgtggcttatatgcccaacatgctcatgtgttgctttgaccagtgtgcaatgtcggacgtaaggccat
ccctctacgtatgaagttggtcttgtcaatgaggcatttccgagttccgagacaaatggcgacagacaagggcaagatggccaattggcaa
ctctttgtgacatgttattgacttacaacggagacaatttgtgcaagattaagtaaaaatgatgtttgagactgtgcaagtggcgagggaataa
cgggtagatccggcgtgctaggcagacaaacttgtatgcctgtctcttcagttggcagctcagttcactcgtgagaggcattgattgaaggg
gcagagcaactcgtgacaggagttgacagcgttggcacctttgcgcccccaacaacatgctcatgaagacgaatgtgccattcctctccgt
ctcagttgcctgtttgatccaagatcctgttttctcgcagttcatgcatataggaggaactcaggaattcgacagacggtcagcaatggcccgt
ttgattggcggatgttattacttgaatcctaatcatgctgtgaccatatcaaccactgcaggtgataagggggatgaacatctccatctcatgtaat
atgagcccaacgactgcaaggcttatataatgcctcaccggtgagcagcaaaggatgatgggatcggcagaacagatggcgggaacatc
aacactcagtggaaattgatgcctccaatgccgcccctgccgggcaatatatactagctgggagagaagcaagaagtcgatcaacgtactc
cggcgtgagccacagcatttgagttaccaatcagagaacgcagggatgtccgctgcccgccctactgctgtacacgtacgagatcctgatg

acttcatgttgtggcggcaaagaagatgcaaggggggtctgccaggttgcgtccgcttggcccatcgcatggagggagactcttggcggct
catttcagctcagccgctgctgtacaacaatcccggcctgctgttggaaggctgtggcaggagtcggctgtgccgaatctcgcccctttgttc
tcccacggcagggcaatttctgggttacctcagtatccctggagttacggaacctctgatgaggactccgatgctactgctctatctgcccgt
gagaagcgggctcgtgtctttgcagaggacagctctctggagagcgagctcgccgacgaagaggctcctaccactgaggtggagattgtt
cttcgagctgtctgggttgagatcctccgggatgtgaaccgaatccgtaagcaggacattggaaagaagcacaacttcttcctgcttggcgg
cgactctctcacaaccatcgagcttgttactgctgcttatcaatacggcattcgtctgagtccggcagccgtatcggacaacgctgagcttgc
ccagatggcagctgtggcaaccatcgaacacgacagcgcaatgatggctgagacaaagcccttcagcttgattagcagtgcccgggcta
gctagttacgcttgtttatttacgacaagatctagaagattcgagatagaataataataacaacaatttgcctcttctttccaccttttcagtctt
actctcccttctgacattgaacgcctcaatcagtcagtcgccttgtacttggcacggtaatcctccgtgttcttgatatcctcaggggtagcaaa
gcccttcatgccatcgataatgtcatccagagtgaggatggcaaagatggggatgccgtactccttcctcagctcgccaatggcactcggtc
caggcttggagtcgtcgccatccgcagcggggagcttctccatgcggtccagggccacgacgatgccggcgacgatgccgcccctccttg
gtgatcttctcaatggcgtccctcttggcggtgccggcggtgatgacgtcgtcgacaatcaggaccctcttgcccttgagcgaagcgccga
cgatgttgccgccctcgccgtggtccttggcctccttgcggtcaaacgagtaggagacgcggtccaggttctggggcgccagctcgccga
gcttgatggtgatggcggagcacagcgggatgcccttgtaggccgggccgaagacgatgtcgaactctaggccggccttctcctgggcct
cgatgatggtctttgcaaaggcggaggcgatggcgccggcgaggcgcgccgtgtggaattcgcccgcgttgaagaagtaggggggatat
ccgcttggacttgagctcgaagctgccaaacttgaggacgccgccgtcgatggcggatttgaggaagtcctgcttgtaggcaggcagctg
ggaggtggtagccattctgttggatttggatagtgtccttattctctgatttgaacagtagatcaggacgagtgagagggatgcagaggttgg
attggagtggttgagctataaaatttagaggcgcgccgtatcgagttttcacatggaagtcaaagcgtacagtgcgagcttgtacgttggtctt
agtatcccacaagcttctgtctaggtatgatgatggctataagtcacccaaggcagaactcatcttgaagattgtctagagtgattttaccgctg
atgaaatgactggactccctcctcctgctcttatacgaaaaattgcctgactctgcaaaggttgtttgtcttggaagatgatgtgcccccccatc
gctcttatctcataccccgccatctttctagattctcatcttcaacaagaggggcaatccatgatctgcgatccagatgtgcttctggcctcatac
tctgccttcaggttgatgttcacttaattggtgacgaattcagctgatttgctgcagtatgctttgtgttggttctttccaggcttgtgccagccatg
agcgctttgagagcatgttgtcacctataaactcgagtaacggccacatattgttcactacttgaatcacatacctaattttgatagaattgacat
gtttaaagagctgaggtagctttaatgcctctgaagtattgtgacacagcttctcacagagtgagaatgaaaagttggactccccctaatgaa
gtaaaagtttcgtctctgaacggtgaagagcatagatccggcatcaactacctggctagactacgacgtcaattctgcggccttttgaccttta
tatatgtccattaatgcaatagattcttttttttttttttttttttttttttttttttttttttgcccaatttcgcagatcaaagtggacgttatagcatcataa
ctaagctcagttgctgagggaagccgtctactaccttagcccatccatccagctccataccttgatactttagacgtgaagcaattcacactgt
acgtctcgcagctctccttcccgctcttgcttccccactggggtccatggtgcgtgtatcgtcccctcccccggggtgtgaggtcaactatcg
cggcgaaaaggcttccgtggcttgctacgtggcgatgcctggccgaaaggacgtagaggccagggagaagggaaatgcctcgacggtt
gagcttgtgtccaagcagtttgaattccgcggaccgaggtgagtagtcttaaatgggctctagttgaagcaacacgcggaggctaaccatc
catactcttcagcctaactattgaggatgctgaagactctgcctcgctggtgacacttgatgaagcctgcaaggctgtcagaaacggcagcg
cccaagccgctctcgttgcgggcatcaacctcatcgccaccggggcgactgcggatgatctcgagggagaagcggttgttgcagtgttca
tcaagccgctgagcgatgcagtccgcgatggaaatcctattcaggccgtcatccgcgctgccagttcggattcgagtgatgctgaagtcgtt
gaagtgagtctcctttccccatccagatggattgtaagatttgaacacggaagaagagtctgtgaaactaacagtggtcatgcagtgttccga

tgtccttatcgctgggagctcctctcagcctgaccagggcgatctgaagagtgcatctggcctcgtcagtctgatcaaagccatggcctcttt
ggagcatcaggacgtcctgtattcagccaccactctatcaaagggtaagaatttgagtcaacagtgtttttgtttgccgccagctgttgttgttgt
caacgtccagcgcttacctaagccagcagatgaaacaccaagccagaagcacctcgaccttccctctggtcgagctcaaagaatccgcat
caacttcgctggaaaatccgggcatgaatctcacttcgtcattgagccgcatcggcagcatgaatccccggcaagcaacgatgctgctggg
ccacagctggtgttgttctctgcaaacaacgtgacgtctctcaaccagcagatgcagcttcaccacggcggccgc

### 1.4. Generation of T. reesei strains with disruptions of tex1 and/or tex2

*T. reesei* strain RL-P37 (Sheir-Neiss and Montenecourt (1984) Appl. Microbiol. Biotechnol. 20:46-53) is grown on PDA plates and allowed to sporulate. Spores are collected and spread onto Vogel's minimal medium agar plates with 2 mg/ml uridine and 0.12% fluoroorotic acid. Colonies that grow on this medium are picked and tested for uridine auxotrophy by inability to grow on Vogel's minimal medium agar plates without added uridine. Genomic DNA is extracted from uridine auxotrophic mutants and the *pyr2* open reading frame is amplified by PCR. DNA sequence analysis of this amplified DNA identifies those mutant strains that have a mutation within the *pyr2* gene. One strain is chosen and designated *T. reesei* RL-P37 *pyr2-.*

Protoplasts of the *T. reesei* RL-P37 *pyr2-* strain are transformed with the *tex1* or *tex2* disruption cassette by PEG-mediated transformation, and plated on Vogel's minimal medium containing 1.2 M sorbitol. *Trichoderma* transformation is described, *e*.*g*., in U.S. Pat. No. 5,246,853. Candidate transformants are transferred to Vogel's minimal medium and, after growth, genomic DNA is isolated. PCR analysis reveals clones in which the *tex1* or *tex2* disruption cassette has integrated by homologous recombination. These clones are designated RL-P37 Deltex1 and RL-P37 Deltex2 respectively.

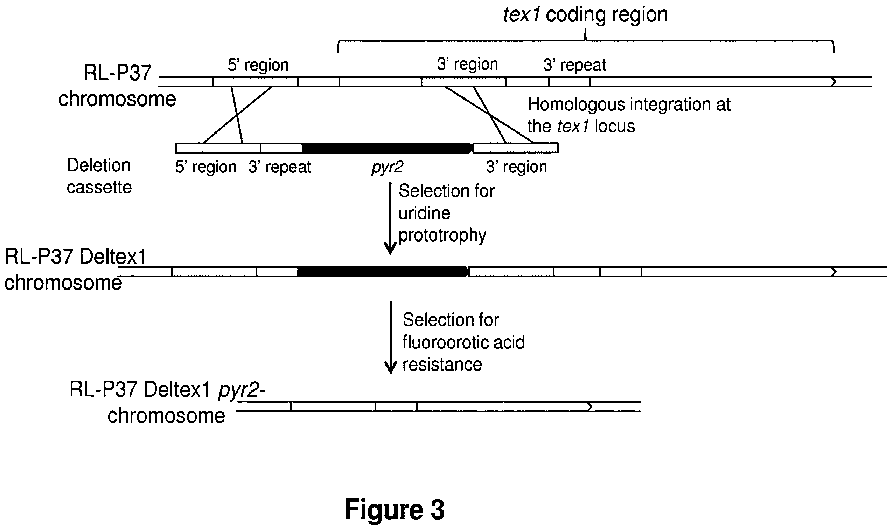

A derivative of strain RL-P37 in which the *tex1* gene has been disrupted is chosen. Spores of this strain are spread onto Vogel's minimal medium with 2 mg/ml uridine and 0.12% fluoroorotic acid. Colonies that grow on this medium are picked and tested for uridine auxotrophy by inability to grow on Vogel's minimal medium agar plates without added uridine. Genomic DNA is extracted from uridine auxotrophic mutants and PCR analysis is conducted to identify auxotrophs in which the *pyr2* gene has been excised from the *tex1* locus by recombination between the 0.5-kb repeated regions of the *tex1* coding region (Figure 3). This strain is designated RL-P37 Deltex1 *pyr2-.* Figure 3 is a diagrammatic representation of the structure of the *T. reesei* chromosome at the *tex1* locus before and after the *tex1* disruption cassette integrates and then after loop-out of *the pyr2* gene upon selection for fluoroorotic acid resistance.

Protoplasts of *T*. *reesei* RL-P37 Deltex1 *pyr2-* are transformed with the *tex2* disruption cassette by PEG-mediated transformation, and plated on Vogel's minimal medium containing sorbitol. *Trichoderma* transformation is described, *e.g.,* in U.S. Pat. No. 5,246,853. Candidate transformants are transferred to Vogel's minimal medium and, after growth, genomic DNA is isolated. PCR analysis reveals clones in which the *tex2* disruption cassette has integrated by homologous recombination. This strain is designated RL-P37 Deltex12.

The reader will appreciate that a similar method can be used to first disrupt the *tex2* gene, followed by disruption of the *tex1* gene, to produce essentially the same variant *T*. *reesei* RL-P37 cells.

### 1.5. Growth of tex1 or tex2-disrupted T. reesei strains in submerged culture and on solid medium

The *tex1* and *tex2*-disrupted RL-P37 strains, along with the RL-P37 parental strain with intact *tex1* and *tex2* genes, are grown under identical conditions on liquid medium (*i*.*e*., a conventional submerged culture medium such as *T*. *reesei* Glycine minimal medium with lactose as carbon source), semi-solid medium (*i.e.,* liquid medium containing insoluble or partially-soluble material, such as a biomass substrate, as carbon source), and solid medium (*i.e.,* agar medium such as PDA), and the production levels of peptaibols are compared. It is expected that cells of the RL-P37 parental strain produce detectable levels of peptaibols when grown on the solid medium and the semi-solid medium but not the liquid medium. In contrast, cells of the *tex1* and *tex2*-disrupted RL-P37 strains produce reduced levels of peptaibols, or do not produce detectable levels of peptaibols, when grown on the solid medium and the semi-solid medium, and, like cells of the parental strain, do not produce peptaibols in liquid medium.

### Example 2. Assays

### 2.1. Detection of peptaibols by MALDI-TOF

The current preferred method for identifying and quantitating peptaibols is detection by matrix-assisted laser desorption/ionization time of flight mass spectrometry (MALDI-TOF). Ascomycetes spp. fungal cells can be harvested from liquid, semi-solid, or solid medium. Where the fungal cells arc harvested from solid medium they may be grown on the surface of a cellophane or other membrane to assist in separating and harvesting the cells from media components. Mycelia can also be separated from media components by centrifugation or filtration, followed, optionally by rinsing.

Harvested, dried mycelia are then resuspended in an aqueous/organic or organic solvent system, such as water/ethanol/acetonitrile (1:1:1), water/acetonitrile/TFA (70:30:0.07), dichloromethane/ethanol 90:10, or similar, which is suitable for analysis by MALDI-TOF. Optionally, peptaibols can be purified prior to detection by MALDI-TOF, *e*.*g*., by binding to a reverse phase column. The references cited, herein, refer to several similar methods to detect peptaibols, and provide MALTI-TOF settings and peptaibol charge and mass reference data that can be used to identify pcptaibols.

### 2.2. Ethanol and Carbohydrate Determinations

Ethanol and carbohydrate composition of samples are determined using an HPLC method. Briefly, a 1.5 mL Eppendorf centrifuge tube was filled with sample, cooled on ice for 10 min., and centrifuged for 1 min in Eppendorf table top centrifuge. A 0.5 mL sample of the supernatant is transferred to a test tube containing 0.05 mL of Kill solution (1.1 N H₂SO₄) and allowed to stand for 5 min. 5.0 mL of water is added to the test tube sample and then filtered into a HPLC vial through 0.45 µm Nylon Syringe Filter; and run on HPLC under one of the following conditions:

(A) Ethanol System: Column: Phenomenex Rezcx Organic Acid Column (RHM-Monosaccharide) #00H 0132-KO (Equivalent to Bio-Rad 87H); Column Temperature: 60°C.; Mobile Phase: 0.01 N H₂SO₄; Flow Rate: 0.6 mL/min; Detector: RI; and Injection Volume: 20 µL. These conditions can be used to detect ethanol.

(B) Carbohydrate System: Column: Phenomenex Rezex Carbohydrate (RCM-Monosaccharidc) #00H-0130-KO (Equivalent to Bio-Rad 87H); Column Temperature: 70°C.; Mobile Phase: Nanopure DI H.sub.20; Flow Rate: 0.8 mL/min; Detector: RI; Injection Volume: 10 µL (3% DS Material). The column separates based on the molecular weight of the saccharides, which are designated as DP-1 (monosaccharides); DP-2 (disaccharides); DP-3 (trisaccharides) and DP>3 (oligosaccharide sugars having a degree of polymerization greater than 3).

### 2.3. Residual starch iodine test

Sample of fermentation broth are centrifuged in 2 ml plastic centrifuge tubes. The supernatant is decanted and the tube containing the pellet is placed in an ice bath. Several drops of 0.025 N iodine solution (0.1 N iodine from VWR Cat. No. VW3207-1 diluted 4 times) was added to the pellet and mixed. A positive (+) starch shows a range of color from blue to purple and the intensity of color is directly proportional to the concentration of starch. A negative result (-) remains yellowish.

### 2.4. Determination of total starch content

The enzyme-enzyme starch liquefaction and saccharification process (dual enzyme method) is used to determine the total starch content. In a typical analysis, 2 g of dry sample is taken in a 100 ml Kohlraucsh flask and 45 ml of MOPS buffer, pH 7.0 are added. The slurry is well stirred for 30 min. SPEZYME FRED (1:50 diluted in water), 1.0 ml is added and the sample is heated to boiling for 3-5 min. The flask is placed in an autoclave maintained at 121°C. for 15 min. After autoclaving the flask is placed in a water bath at 95°C and 1 ml of 1:50 diluted SPEZYME FRED is added and incubated for 45 min. The pH is adjusted to 4.2 and the temperature is reduced to 60°C. This is followed by addition of 20 ml acetate buffer, pH 4.2. Saccharification is carried out by adding 1.0 ml of 1:100 diluted OPTIDEX L-400 (Glucoamylase from Danisco US Inc.) and the incubation is continued for 18 hr at 60°C. The enzyme reaction is terminated by heating at 95°C for 10 min. The total sugar composition is determined by HPLC analysis using glucose as a standard. The soluble starch hydrolysate from water extraction of a sample at room temperature without enzymatic treatment was subtracted from the total sugar.

### 2.4. Total protein analysis

The total nitrogen (N) in sample preparations is determined using the Kjeldhal method (American Assoc. Cereal Chemists (AACC) (1983) Methods 22B60 8th Ed. St Paul, Minn.). Protein content is calculated as 6.25 times total N.

### Example 3. Growth of a fermenting organism in the presence and absence of peptaibols

A *Trichoderma reesei* strain derived from RL-P37 in which the *tex1* and *tex2* genes are disrupted and the parental RL-P37 strain in which these two genes are not disrupted are grown in YEG culture medium (yeast extract 5 g/L, glucose 20 g/L) for two days at 28°C in a shake flask. An aliquot from this initial culture is used to inoculate liquid medium with 2% w/v insoluble biomass as the carbon source. The insoluble biomass may be wheat bran, beet pulp, pretreated corn cob, or pretreated corn stover, among other substrates. Many pretreatment methods are known in the art, including the use of dilute ammonia.

After a period of culture (*e.g.,* 5 days) at 28°C in a shake flask the cells may be inactivated using a method such as that disclosed in US Patent No. 5,801,034. The levels of peptaibols produced by the *Trichoderma* cells with disrupted *tex1* and *tex2* genes is measured and compared to the levels of peptaibols produced by the parental *Trichoderma* cells. Whereas the parental cells produce peptaibols when grown on insoluble biomass, the cells with disrupted *tex1* and *tex2* genes produce no detectable peptaibols.

Either the whole culture broth with live cells or the culture broth with inactivated cells from the parental cells or the cells with disrupted *tex1* and *tex2* genes is used for saccharification of a pretreated biomass suspension as follows. In a enzymatic hydrolysis step, a suitable lignocellulosic biomass feedstock, for example, one derived from an agricultural crop, a byproduct of a food or feed production, a lignocellulosic waste product, a plant residue, or a waste paper or waste paper product, pretreated using various methods known in the art can be loaded into a suitable reactor followed by the addition of the whole culture broth with live cells or the culture broth with inactivated cells from the parental cells or the cells with disrupted *tex1* and *tex2* genes, at a certain solids loading level (typically below about 40%). A mixing or agitation mechanism is applied to the reactor to achieve a reaction slurry, preferably one with high homogeneity. The entirety of this enzymatic hydrolysis step can take place in a simultaneous saccharification and fermentation fashion, wherein the enzymatic hydrolysis of the pretreated biomass substrate occurs in the presence of a suitable ethanologen, for example, a yeast or a *Zymomonas mobilis,* and whereby the sugars produced by the saccharification or hydrolysis of the pretreated biomass materials in the suspension arc continuously or simultaneously fermented into ethanol and other byproducts in the same reactor.

In a first alternative, the conversion of the biomass substrate to ethanol can take place in a separate saccharification and fermentation fashion, wherein the entirety of the enzymatic hydrolysis or saccharification step can take place in the absence of the ethanologen, whereby the glucan and xylan in the pretreated biomass substrate are first enzymatically hydrolysed into fermentable sugars. The hydrolysate is then subject to the suitable ethanologen, optionally in a different fermentor, where the fermentable sugars are converted into ethanol and other products.

In a second alternative, the conversion of the biomass substrate into ethanol can take place in a hybrid saccharification and fermentation fashion, wherein the enzymatic hydrolysis step can take place in a first stage, for example, for a period of about 1 to about 60 hours, in the absence of the ethanologen. The ethanologen is then introduced into the reactor whereby ethanol is produced from the fermentable sugars produced in the first stage as well as those that are being continuously produced after the introduction of the ethanolgen. At a preselected time following the start of the fermentation step, the levels of ethanol produced by the ethanologen arc measured.

The levels of ethanol produced in biomass substrate contacted with culture broth from *Trichoderma* cells with disrupted *tex1* and *tex2* genes is higher than the levels of ethanol produced in biomass substrate contacted with culture broth from the parental *Trichoderma* cells, demonstrating that the fermenting organism grows better in the absence of peptaibols.

### Example 4. Growth of a fermenting organism in the presence and absence of peptaibols

A *Trichoderma reesei* strain derived from RL-P37 is constructed in which genes encoding the major secreted cellulases are deleted and the native *Trichoderma* glucoamylase gene is overexpressed as exemplified in U.S. Patent No. 7,413,887. A further variant of this strain is made in which the *tex1* and *tex2* genes are disrupted. The strain in which the *tex1* and *tex2* genes are disrupted and the parental glucoamylase strain in which these two genes are not disrupted are grown in YEG culture medium (yeast extract 5 g/L, glucose 20 g/L) for two days at 28°C in a shake flask. An aliquot from this initial culture is used to inoculate liquid medium with 2% w/v insoluble biomass as the carbon source. The insoluble biomass may be, *e.g.,* granular starch.

After a period of culture (*e.g.,* 5 days) at 28°C in a shake flask the cells may be inactivated using a method such as that disclosed in U.S. Patent No. 5,801,034. The levels of peptaibols produced by the *Trichoderma* cells with disrupted *tex1* and *tex2* genes is measured and compared to the levels of peptaibols produced by the parental *Trichoderma* cells. Whereas the parental cells produce peptaibols when grown on insoluble biomass, the cells with disrupted *tex1* and *tex2* genes produce no detectable peptaibols.

Either the whole culture broth with live cells or the culture broth with inactivated cells from the parental cells or the cells with disrupted *tex1* and *tex2* genes is used for saccharification of a liquefied starch preparation, along with a sufficient amount of α-amylase and a suitable fermenting organism, such as *Saccharomyces cerevisiae* (see, *e.g.,* U.S. Patent Pub. No. 20110318803). At a preselected time following saccharification, the levels of ethanol produced by the fermenting organism are measured. The levels of ethanol produced in a liquefied starch substrate contacted with culture broth from *Trichoderma* cells with disrupted *tex1* and *tex2* genes is higher than the levels of ethanol produced in a liquefied starch substrate contacted with culture broth from the parental *Trichoderma* cells, demonstrating that the fermenting organism grows better in the absence of peptaibols.

## Claims

1. A method of producing a secreted protein of interest in Ascomycete fungal cells grown in a medium that includes insoluble material, comprising: expressing the secreted protein of interest from variant Ascomycete fungal cells comprising a genetic modification to introduce a gene comprising the protein of interest and further comprising a genetic modification that disrupts at least one non-ribosomal peptide synthetase (NRPS), wherein the variant cells produce a reduced amount of peptaibols relative to the amount of peptaibols produced by equivalent, non-modified Ascomycete fungal cells when grown in a medium comprising insoluble material.

2. The method of claim 1, wherein the medium including insoluble material comprises a carbon source provided in the form of a semi-solid suspension.

3. The method of claim 1 or claim 2, wherein the medium is a biomass substrate.

4. The method of any one of the preceding claims, which is a method of increasing the production of said protein of interest compared to an Ascomycete fungal cell not having the genetic modification that disrupts at least one non-ribosomal peptide synthetase (NRPS).

5. A method for producing a protein of interest in Ascomycete fungal cells, comprising:
expressing the protein of interest in variant Ascomycete fungal cells comprising a genetic modification that reduces the production of peptaibols relative to the amount of peptaibols produced by equivalent, non-modified Ascomycete fungal cells; wherein the genetic modification disrupts at least one non-ribosomal peptide synthase (NRPS); wherein the reduced production of peptaibols results in improved growth of the variant Ascomycete fungal cells compared to the non-modified Ascomycete fungal cells; improved growth of a second microbial organism grown in the presence of the variant Ascomycete fungal cells compared to the growth of the second microbial organism grown in the presence of equivalent non-modified Ascomycete fungal cells; or improved growth of the second microbial organism grown in the presence of the protein of interest produced by the variant Ascomycete fungal cells, compared to growth of the second microbial organism grown in the presence of a protein of interest produced by equivalent non-modified Ascomycete fungal cells, wherein the variant Ascomycete fungal cells are grown on a carbon source that includes insoluble material.

6. The method of claim 5, wherein the variant Ascomycete fungal cells are grown on a carbon source provided in the form of a semi-solid suspension.

7. The method of claim 5 or 6, wherein the protein of interest is a secreted protein.

8. A method for improving the conversion of a biomass substrate into a fermented end-product, or intermediate, thereof, comprising contacting the biomass substrate with variant Ascomycete fungal cells producing enzymes for hydrolyzing the biomass substrate into sugars for use by cells of a second, fermenting organism capable of fermenting the sugars into end products, or intermediates, thereof, the variant Ascomycete fungal cells comprising a genetic modification that disrupts at least one non-ribosomal peptide synthase (NRPS) to reduce the production of peptaibols relative to the amount produced by equivalent, non-modified Ascomycete fungal cells, wherein the reduced production of peptaibols by the variant Ascomycete fungal cells improves the yield of end products, or intermediates, thereof, produced by the cells of the second organism compared to the amount of end products, or intermediates, thereof, produced by the cells of the second organism from fermentable sugars produced the equivalent, non-modified Ascomycete cells.

9. The method of claim 8, wherein the fermenting organism is a bacteria or yeast.

10. The method of claim 8 or 9, wherein the fermenting organism is a yeast.

11. The method of any one of claims 8 to 10, wherein the fermenting organism is selected from the group consisting of a *Saccharomyces* spp. and a *Zymomonas* spp.

12. The method of any one of claims 8.to 11, wherein the fermenting organism capable of fermenting the sugars is genetically modified to be capable of utilizing both six-carbon sugars and five-carbon sugars.

13. The method of any one of the preceding claims, wherein:
(a) the genetic modification to reduce the production of peptaibols is the disruption of at least one non-ribosomal peptide synthetase (NRPS) gene without the introduction of foreign DNA; or
(b) the genetic modification to reduce the production of peptaibols is the complete or partial deletion of at least one non-ribosomal peptide synthetase (NRPS) gene.

14. The method of any one of the preceding claims, wherein the non-ribosomal peptide synthetase (NRPS) gene is *tex1* or *tex2.*

15. The method of any one of the preceding claims, wherein:
(a) the Ascomycete fungal cells are filamentous fungi cells in the subphylum Pezizomycotina; or
(b) the Ascomycete fungal cells are *Trichoderma* spp. Cells.

16. The method of any preceding claims, wherein the Ascomycete fungal cells are *Trichoderma reesei* cells.

17. The method of claim 16 wherein the *Trichoderma reesei* cells comprise genetic modifications that disrupt the tex 1 and tex2 gene.

## Patentansprüche

1. Verfahren zum Herstellen eines sezernierten Proteins von Interesse in Ascomycet-Pilzzellen, die in einem Medium gezüchtet werden, das unlösliches Material einschließt, umfassend: Exprimieren des sezernierten Proteins von Interesse aus verschiedenen Ascomycet-Pilzzellen, die eine genetische Modifikation umfassen, um ein Gen einzuführen, das das Protein von Interesse umfasst, und ferner umfassend eine genetische Modifikation, die mindestens eine nichtribosomale Peptidsynthetase (NRPS) unterbricht, wobei die verschiedenen Zellen, bezogen auf die Menge von Peptaibolen, die von äquivalenten, nicht modifizierten Ascomycet-Pilzzellen erzeugt werden, wenn sie in einem unlösliches Material umfassenden Medium gezüchtet werden, eine reduzierte Menge von Peptaibolen erzeugen.

2. Verfahren gemäß Anspruch 1, wobei das Medium, das unlösliches Material einschließt, eine Kohlenstoffquelle umfasst, die in der Form einer halbfesten Suspension bereitgestellt wird.

3. Verfahren gemäß Anspruch 1 oder Anspruch 2, wobei das Medium ein Biomassesubstrat ist.

4. Verfahren gemäß einem der vorstehenden Ansprüche, das ein Verfahren zur Steigerung der Produktion des Proteins von Interesse im Vergleich zu einer Ascomycet-Pilzzelle ist, die die genetische Modifikation, die mindestens eine nichtribosomale Peptidsynthetase (NRPS) unterbricht, nicht umfasst.

5. Verfahren zum Herstellen eines Proteins von Interesse in Ascomycet-Pilzzellen, umfassend: Exprimieren des Proteins von Interesse in verschiedenen Ascomycet-Pilzzellen, die eine genetische Modifikation umfassen, die die Produktion von Peptaibolen, bezogen auf die Menge von Peptaibolen, die von äquivalenten, nicht modifizierten Ascomycet-Pilzzellen erzeugt werden, reduziert; wobei die genetische Modifikation mindestens eine nichtribosomale Peptidsynthetase (NRPS) unterbricht; wobei die reduzierte Produktion von Peptaibolen zu verbessertem Wachstum der verschiedenen Ascomycet-Pilzzellen im Vergleich zu den nicht modifizierten Ascomycet-Pilzzellen; verbessertem Wachstum eines zweiten mikrobiellen Organismus, der in Gegenwart der verschiedenen Ascomycet-Pilzzellen gezüchtet wird, im Vergleich zu dem Wachstum des zweiten mikrobiellen Organismus, der in Gegenwart äquivalenter nicht modifizierter Ascomycet-Pilzzellen gezüchtet wird; oder verbessertem Wachstum des zweiten mikrobiellen Organismus, der in Gegenwart des Proteins von Interesse gezüchtet wird, das durch die verschiedenen Ascomycet-Pilzzellen erzeugt wurde, im Vergleich zu dem Wachstum des zweiten mikrobiellen Organismus, der in Gegenwart eines Proteins von Interesse gezüchtet wird, das durch äquivalente nicht modifizierte Ascomycet-Pilzzellen erzeugt wurde, führt, wobei die verschiedenen Ascomycet-Pilzzellen auf einer Kohlenstoffquelle gezüchtet werden, die unlösliches Material einschließt.

6. Verfahren gemäß Anspruch 5, wobei die verschiedenen Ascomycet-Pilzzellen auf einer Kohlenstoffquelle gezüchtet werden, die in der Form einer halbfesten Suspension bereitgestellt wird.

7. Verfahren gemäß Anspruch 5 oder 6, wobei das Protein von Interesse ein sezerniertes Protein ist.

8. Verfahren zum Verbessern der Umwandlung eines Biomassesubstrats in ein fermentiertes Endprodukt oder Zwischenprodukt davon, umfassend das Inkontaktbringen des Biomassesubstrats mit verschiedenen Ascomycet-Pilzzellen, die Enzyme zum Hydrolysieren des Biomassesubstrats zu Zuckern zum Gebrauch durch Zellen eines zweiten, fermentierenden Organismus produzieren, der in der Lage ist, die Zucker zu Endprodukten oder Zwischenprodukten davon zu fermentieren, wobei die verschiedenen Ascomycet-Pilzzellen eine genetische Modifikation umfassen, die mindestens eine nichtribosomale Peptidsynthetase (NRPS) unterbricht, um die Produktion von Peptaibolen, bezogen auf die Menge, die von äquivalenten, nicht modifizierten Ascomycet-Pilzzellen erzeugt wird, zu reduzieren, wobei die reduzierte Produktion von Peptaibolen durch die verschiedenen Ascomycet-Pilzzellen die Ausbeute von Endprodukten oder Zwischenprodukten davon, die durch die Zellen des zweiten Organismus erzeugt werden, im Vergleich zu der Menge von Endprodukten oder Zwischenprodukten davon, die von den Zellen des zweiten Organismus aus fermentierbaren Zuckern, die durch die äquivalenten, nicht modifizierten Ascomycet-Zellen produziert werden, verbessert.

9. Verfahren gemäß Anspruch 8, wobei der fermentierende Organismus ein Bakterium oder eine Hefe ist.

10. Verfahren gemäß Anspruch 8 oder 9, wobei der fermentierende Organismus eine Hefe ist.

11. Verfahren gemäß einem der Ansprüche 8 bis 10, wobei der fermentierende Organismus ausgewählt ist aus der Gruppe, bestehend aus *Saccharomyces* spp. und *Zymomonas* spp.

12. Verfahren gemäß einem der Ansprüche 8 bis 11, wobei der fermentierende Organismus, der zum Fermentieren der Zucker in der Lage ist, genetisch modifiziert ist, um sowohl Zucker mit sechs Kohlenstoffen als auch Zucker mit fünf Kohlenstoffen nutzen zu können.

13. Verfahren gemäß einem der vorstehenden Ansprüche, wobei:
(a) die genetische Modifikation zum Reduzieren der Produktion von Peptaibolen die Unterbrechung von mindestens einem Gen einer nichtribosomalen Peptidsynthetase (NRPS) ohne die Einführung fremder DNA ist; oder
(b) die genetische Modifikation zum Reduzieren der Produktion von Peptaibolen die vollständige oder partielle Löschung von mindestens einem Gen einer nichtribosomalen Peptidsynthetase (NRPS) ist.

14. Verfahren gemäß einem der vorstehenden Ansprüche, wobei das Gen der nichtribosomalen Peptidsynthetase (NRPS) *tex1* oder *tex2* ist.

15. Verfahren gemäß einem der vorstehenden Ansprüche, wobei:
(a) die Ascomycet-Pilzzellen fadenförmige Pilzzellen im Unterstamm Pezizomycotina sind; oder
(b) die Ascomycet-Pilzzellen *Trichoderma-*spp.-Zellen sind.

16. Verfahren gemäß einem der vorstehenden Ansprüche, wobei die Ascomycet-Pilzzellen *Trichoderma-reesei-*Zellen sind.

17. Verfahren nach Anspruch 16, wobei die *Trichoderma-reesei-*Zellen genetische Modifikationen umfassen, die das tex1- und das tex2-Gen unterbrechen.

## Revendications

1. Méthode pour la production d'une protéine d'intérêt sécrétée dans des cellules fongiques d'ascomycète qui croissent dans un milieu qui inclut un matériau insoluble, comprenant: l'expression de la protéine d'intérêt sécrétée à partir de cellules fongiques d'ascomycète variantes comprenant une modification génétique pour introduire un gène comprenant la protéine d'intérêt, et comprenant en outre une modification génétique qui perturbe au moins une peptide synthétase non ribosomale (NRPS), dans laquelle les cellules variantes produisent une quantité réduite de peptaibols relativement à la quantité de peptaibols produite par des cellules fongiques d'ascomycète non modifiées, équivalentes lorsqu'elles croissent dans un milieu comprenant un matériau insoluble.

2. Méthode selon la revendication 1, dans laquelle le milieu incluant un matériau insoluble comprend une source de carbone fournie sous la forme d'une suspension semi-solide.

3. Méthode selon la revendication 1 ou la revendication 2, dans laquelle le milieu est un substrat de biomasse.

4. Méthode selon l'une quelconque des revendications précédentes qui est une méthode d'augmentation de la production de ladite protéine d'intérêt comparée à une cellule fongique d'ascomycète n'ayant pas la modification génétique qui perturbe au moins une peptide synthétase non ribosomale (NRPS).

5. Méthode pour la production d'une protéine d'intérêt dans des cellules fongiques d'ascomycète, comprenant: l'expression de la protéine d'intérêt dans des cellules fongiques d'ascomycète variantes comprenant une modification génétique qui réduit la production de peptaibols relativement à la quantité de peptaibols produite par des cellules fongiques d'ascomycète non modifiées, équivalentes; dans laquelle la modification génétique perturbe au moins une peptide synthase non ribosomale (NRPS); dans laquelle la production réduite de peptaibols conduit à une croissance améliorée des cellules fongiques d'ascomycète variantes comparées aux cellules fongiques d'ascomycète non modifiées; la croissance améliorée d'un deuxième organisme microbien qui croît en présence des cellules fongiques d'ascomycète variantes comparée à la croissance du deuxième organisme microbien qui croît en présence des cellules fongiques d'ascomycète non modifiées équivalentes; ou la croissance améliorée du deuxième organisme microbien qui croît en présence de la protéine d'intérêt produite par les cellules fongiques d'ascomycète variantes, comparée à la croissance du deuxième organisme microbien qui croît en présence d'une protéine d'intérêt produite par des cellules fongiques d'ascomycète non modifiées équivalentes, où les cellules fongiques d'ascomycète variantes croissent sur une source de carbone qui inclut un matériau insoluble.

6. Méthode selon la revendication 5, dans laquelle les cellules fongiques d'ascomycète variantes croissent sur une source de carbone fournie sous la forme d'une suspension semi-solide.

7. Méthode selon les revendications 5 ou 6, dans laquelle la protéine d'intérêt est une protéine sécrétée.

8. Méthode pour l'amélioration de la conversion d'un substrat de biomasse dans un produit final fermenté ou un intermédiaire de celui-ci, comprenant la mise en contact du substrat de biomasse avec des cellules fongiques d'ascomycète variantes produisant des enzymes pour l'hydrolyse du substrat de biomasse en sucres pour une utilisation par des cellules d'un deuxième organisme fermenteur capable de fermenter les sucres en produits finaux ou intermédiaires de ceux-ci, les cellules fongiques d'ascomycète variantes comprenant une modification génétique qui perturbe au moins une peptide synthase non ribosomale (NRPS) pour réduire la production de peptaibols relativement à la quantité produite par des cellules fongiques d'ascomycète non modifiées, équivalentes, dans laquelle la production réduite de peptaibols par les cellules fongiques d'ascomycète variantes améliore le rendement de produits finaux ou intermédiaires de ceux-ci, produits par les cellules du deuxième organisme, comparé à la quantité de produits finaux ou intermédiaires de ceux-ci produits par les cellules du deuxième organisme à partir de sucres fermentables produits par les cellules fongiques d'ascomycète non modifiées, équivalentes.

9. Méthode selon la revendication 8, dans laquelle l'organisme fermenteur est une bactérie ou une levure.

10. Méthode selon les revendications 8 ou 9, dans laquelle l'organisme fermenteur est une levure.

11. Méthode selon l'une quelconque des revendications 8 à 10, dans laquelle l'organisme fermenteur est choisi parmi le groupe constitué de *Saccharomyces* spp. et de *Zymomonas* spp.

12. Méthode selon l'une quelconque des revendications 8 à 11, dans laquelle l'organisme fermenteur capable de fermenter les sucres est génétiquement modifié pour être capable d'utiliser à la fois des sucres à six carbones et des sucres à cinq carbones.

13. Méthode selon l'une quelconque des revendications précédentes, dans laquelle :
(a) la modification génétique pour réduire la production de peptaibols est la perturbation d'au moins un gène de peptide synthétase non ribosomale (NRPS) sans l'introduction d'un ADN étranger; ou
(b) la modification génétique pour réduire la production de peptaibols est la délétion complète ou partielle d'au moins un gène de peptide synthétase non ribosomale (NRPS).

14. Méthode selon l'une quelconque des revendications précédentes, dans laquelle le gène de peptide synthétase non ribosomale (NRPS) est *tex1* ou *tex2.*

15. Méthode selon l'une quelconque des revendications précédentes, dans laquelle:
(a) les cellules fongiques d'ascomycète sont des cellules de champignon filamenteux dans le sous-phylum Pezizomycotina; ou
(b) les cellules fongiques d'ascomycète sont des cellules de *Trichoderma* spp.

16. Méthode selon l'une quelconque des revendications précédentes, dans laquelle les cellules fongiques d'ascomycète sont des cellules de *Trichoderma reesei.*

17. Méthode selon la revendication 16, dans laquelle les cellules de *Trichoderma reesei* comprennent des modifications génétiques qui perturbent les gènes tex1 et tex2.
